# EUROPEAN PATENT APPLICATION

(11) **EP 3 950 945 A1**
(43) Date of publication of application: **09.02.2022**
(21) Application number: 19901853.2
(22) Date of filing: 30.12.2019
(51) Int. Cl.: C12N 15/11, C12Q 1/6886

(54) **METHYLATION MODIFICATION-BASED TUMOR MARKER STAMP-EP4**

(30) Priority: 29.12.2018 CN 201811634286
(71) Applicant: Shanghai Epiprobe Biotechnology Co., Ltd., Shanghai 200233 (CN)
(72) Inventor: LI, Zhenyan, Shanghai 200233 (CN); DONG, Shihua, Shanghai 200233 (CN)
(74) Representative: IPLodge bv
(86) International application number: PCT/CN2019/129827
(87) International publication number: WO 2020/135861

(57) **Abstract**

The present invention relates to the field of disease diagnosis markers, and provides a methylation tumor marker STAMP-EP4, and an application thereof. The present invention provides an application of the methylation tumor marker STAMP-EP4 in preparing a cancer diagnosis reagent.

## Description

### FIELD OF DISCLOSURE

The disclosure is in the field of disease diagnostic markers. More specifically, the disclosure relates to a methylation based tumor marker STAMP, Specific Tumor Aligned Methylation of Pan-cancer.

### BACKGROUND OF DISCLOSURE

Tumors have been considered a genetic disease for decades. Several large-scale systematic sequencings for human have confirmed that the number of somatic mutations in cancer tissues is significantly less than expected. These results suggest that cancer is not a simple genetic disease.

In order to diagnose tumor, many new tumor markers have been discovered and used for clinical diagnosis in recent years. Before 1980, tumor markers were mainly hormones, enzymes, proteins and other cell secretions, such as carcinoembryonic antigen (CEA) and alpha fetoprotein (AFP) used as markers of gastric cancer, liver cancer and other tumors, carbohydrate antigen 125 (CA125) used as a marker of cervical cancer, and prostate specific antigen (PSA) used as a marker of prostate cancer. Although these tumor markers are still used in clinic, their sensitivity and accuracy have been difficult to meet the clinical needs.

More and more evidences show that small changes in epigenetic regulation play an important role in tumors. Epigenetics is a subject that studies that the heritable change of gene function without a change of DNA sequence, which eventually leads to the change of phenotype. Epigenetics mainly includes DNA methylation, Histone modification, microRNA level changes and other biochemical processes. DNA methylation is one of the epigenetic mechanisms, refers to the process of transferring methyl from S-adenosylmethionine (methyl donor) to specific bases under the catalysis of DNA methyltransferase (DMT). However, DNA methylation in mammals mainly occurs at the C of 5'-CpG-3', which results in 5-methylcytosine (5mC).

Fluid biopsy is a technique for the diagnosis and prediction of tumors using circulating tumor cells or circulating tumor DNA as detection targets. The technology has many shortcomings. First, the sensitivity and specificity are not good enough. The tumor itself is heterogeneous, including a variety of subtypes of cell populations. The proportion of tumor DNA in clinical samples, especially blood samples, is very low. The existing tumor markers are difficult to meet the sensitivity of clinical requirements, and it is easy to cause misdiagnosis. Second, one marker has good effect only for one or a few kinds of tumors. As the DNA sources in blood are very complex, the existing tumor markers cannot solve the complex problems of tumor source and metastasis. Because of these complexities, it is difficult for many DNA methylation tumor markers to have a unified standard in clinical application, which seriously affects the sensitivity and accuracy of the markers.

In summary, in the field of tumor diagnosis, it is urgent to discover and study more new tumor markers, so as to provide more possibilities for tumor diagnosis.

### SUMMARY OF DISCLOSURE

The object of the disclosure is to provide a method for detecting tumor based on abnormal hypermethylation of specific sites in tumor using DNA methylation modification as a tumor marker.

The first aspect of the present disclosure provides an isolated polynucleotide, including: (a) a polynucleotide with a nucleotide sequence as shown in SEQ ID NO: 1; (b) (a) a polynucleotide with a nucleotide sequence as shown in SEQ ID NO: 2; (c) a fragment of the polynucleotide of (a)-(b), having at least one (such as 2-54 or 2-204, more specifically 3, 5, 10, 15, 20, 25, 30, 35, 40, 45, 50, 60, 80, 100, 120, 140, 160, 180, 200) CpG site with modification; and/or (d) a nucleic acid (such as the polynucleotide with a nucleotide sequence as shown in SEQ ID NO: 5 or 6) complementary to the polynucleotide or fragment of (a)-(c).

In a preferable embodiment, the modification includes 5-methylation(5mC), 5-hydroxymethylation(5hmC), 5-formylcytosine(5fC) or 5-carboxylcytosine(5-caC).

The second aspect of the disclosure provides an isolated polynucleotide, which is converted from the polynucleotide of the first aspect, and as compared with the sequence of the first aspect, the cytosine C of the CpG site(s) with modification is unchanged, and the unmodified cytosine is converted into T or U.

In a preferable embodiment, it is converted from the polynucleotide corresponding to the first aspect by bisulfite treatment. In another preferable embodiment, the polynucleotide includes: (e) a polynucleotide with a nucleotide sequence as shown in SEQ ID NO: 3 or 7; (f) a polynucleotide with a nucleotide sequence as shown in SEQ ID NO: 4 or 8; (g) a fragment of the polynucleotide of (e)-(f), having at least one (such as 2-54 or 2-204, more specifically 3, 5, 10, 15, 20, 25, 30, 35, 40, 45, 50, 60, 80, 100, 120, 140, 160, 180, 200) CpG site with modification.

The third aspect of the disclosure provides a use of the polynucleotide described in the first or second aspect in manufacture of a tumor detection agent or kit.

In a preferable embodiment, the tumors include (but are not limited to): hematologic cancers such as leukemia, lymphoma, multiple myeloma; digestive system tumors such as esophageal cancer (cancer of the esophagus), gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; gynecological and reproductive system tumors such as breast cancer, ovarian cancer, cervical cancer, vulvar cancer, testicular cancer, prostate cancer, penile cancer; urinary system tumors such as kidney cancer, bladder cancer, skin and other systems tumors such as skin cancer, melanoma, osteosarcoma, liposarcoma, thyroid cancer.

In another preferable embodiment, samples of the tumor include: tissue samples, paraffin embedded samples, blood samples, pleural effusion samples, alveolar lavage fluid samples, ascites and lavage fluid samples, bile samples, stool samples, urine samples, saliva samples, sputum samples, cerebrospinal fluid samples, cell smear samples, cervical scraping or brushing samples, tissue and cell biopsy samples.

The fourth aspect of the disclosure provides a method of preparing a tumor detection agent, including: providing the polynucleotide described in the first or second aspect, designing a detection agent for specifically detecting the modification on CPG site(s) of a target sequence which is the full length or fragment of the polynucleotide; wherein, the target sequence has at least one (such as 2-54 or 2-204, more specifically 3, 5, 10, 15, 20, 25, 30, 35, 40, 45, 50, 60, 80, 100, 120, 140, 160, 180, 200) modified CpG site; preferably, the detection agent includes (but is not limited to) primers or probes.

The fifth aspect of the disclosure provides an agent or a combination of agents which specifically detect the modification on CPG site(s) of a target sequence, which is the full length or fragment of any of the polynucleotides described in the first or second aspect and has at least one (such as 2-54 or 2-204, more specifically 3, 5, 10, 15, 20, 25, 30, 35, 40, 45, 50, 60, 80, 100, 120, 140, 160, 180, 200) modified CpG site.

In a preferable embodiment, the agent or combination of agents is for a gene sequence containing the target sequence (designed based on the gene sequence), and the gene sequence includes gene Panels or gene groups.

In another preferable embodiment, the detection agent comprises: primers or probes. In another preferable embodiment, the primers are selected from the group consisting of: the primers shown in SEQ ID NO: 9 and 10; the primers shown in SEQ ID NO: 11 and
12; the primers shown in SEQ ID NO: 13 and 14; the primers shown in SEQ ID NO: 15 and 16; or the primers shown in SEQ ID NO: 17 and 18.

In the sixth aspect of the disclosure, a use of the agent or combination of agents described in the fifth aspect of the disclosure in the manufacture of a kit for detecting tumors is provided; preferably, the tumors include (but are not limited to): digestive system tumors such as esophageal cancer (cancer of the esophagus), gastric cancer, colorectal cancer, liver cancer, pancreatic cancer, bile duct and gallbladder cancer; respiratory system tumors such as lung cancer, pleuroma; hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

The seventh aspect of the present disclosure provides a detection kit, comprising container(s) and the agent or combination of agents described above in the container(s); preferably, each agent is placed in an independent container.

In another preferable embodiment, the kit also includes: bisulfite, DNA purification agent, DNA extraction agent, PCR amplification agent and/or instruction for use (indicating operation steps of the detection and a result judgment standard).

In the eighth aspect of the disclosure, a method for detecting the methylation profile of a sample *in vitro* is provided, including: (i) providing the sample and extracting the nucleic acid; (ii) detecting the modification on CPG site(s) of a target sequence in the nucleic acid of (i), wherein the target sequence is the polynucleotide described in the first aspect or the polynucleotide converted therefrom as described in the second aspect.

In a preferable embodiment, in step (3), the analysis methods include pyrosequencing, bisulfite conversion sequencing, method using methylation chip, qPCR, digital PCR, second generation sequencing, third generation sequencing, whole genome methylation sequencing, DNA enrichment detection, simplified bisulfite sequencing technology, HPLC, MassArray, methylation specific PCR , or their combination, as well as *in vitro* detection and *in vivo* tracer detection for the combined gene group of partial or all of the methylation sites in the sequence of the disclosure. In addition, other methylation detection methods and newly developed methylation detection methods in the future can be applied to the disclosure.

In another preferable embodiment, step (ii) includes: (1) treating the product of (i) to convert the unmodified cytosine into uracil; preferably, the modification includes 5-methylation(5mC), 5-hydroxymethylation(5hmC), 5-formylcytosine(5fC) or 5-carboxylcytosine(5-caC); preferably, treating the nucleic acid of step (i) with bisulfite; and (2) analyzing the modification on CPG site(s) of the target sequence in the nucleic acid treated by (1).

In another preferable embodiment, the abnormal methylation profile is the high level of methylation of C in CPG(s) of the polynucleotide.

In another preferable embodiment, the methylation profile detecting method is not for the purpose of directly obtaining the diagnosis result of a disease, or is not a diagnostic method.

The ninth aspect of the disclosure provides a tumor diagnosis kit, including a primer pair designed based on the sequence described in the first or second aspect of the disclosure, and gene Panels or gene groups containing the sequence, to obtain the characteristics of normal cells and tumor cells through DNA methylation detection.

Other aspects of the disclosure will be apparent to those skilled in the art based on the disclosure herein.

### BRIEF DESCRIPTION OF DRAWINGS

Figure 1. Difference in methylation levels of 001-054 methylation sites of SEQ ID NO: 1 in normal liver cells and liver cancer cells.
Figure 2. Difference in methylation levels of 001-044 methylation sites of SEQ ID NO:2 in normal liver cells and liver cancer cells.
Figure 3. Difference in methylation levels of 045-125 methylation sites of SEQ ID NO:2 in normal liver cells and liver cancer cells.
Figure 4. Difference in methylation levels of 126-204 methylation sites of SEQ ID NO:2 in normal liver cells and liver cancer cells.
Figure 5. In clinical samples of breast cancer, the methylation value of STAMP-EP4 in the experimental group was significantly higher than that of paracancerous tissues; p<0.001.
Figure 6. In clinical samples of leukemia, the methylation value of STAMP-EP4 in the experimental group was significantly higher than that of non-cancer tissues; p<0.01.
Figure 7. In clinical samples of colorectal cancer, the methylation value of STAMP-EP4 in the experimental group was significantly higher than that of paracancerous tissues; p<0.001.
Figure 8. In clinical samples of liver cancer, the methylation value of STAMP-EP4 in the experimental group was significantly higher than that of paracancerous tissues; p<0.01.
Figure 9. In clinical samples of lung cancer, the methylation value of STAMP-EP4 in the experimental group was significantly higher than that of paracancerous tissues; p<0.01.
Figure 10. In clinical samples of pancreatic cancer, the methylation value of STAMP-EP4 in the experimental group was significantly higher than that of paracancerous tissues; p<0.01.
Figure 11. In clinical samples of esophageal cancer, the methylation value of STAMP-EP4 in the experimental group was significantly higher than that of paracancerous tissues; p<0.01.

### DETAILED DESCRIPTION

The inventor is committed to the research of tumor markers. After extensive research and screening, the inventor provides a universal DNA methylation tumor marker, STAMP (Specific Tumor Aligned Methylation of Pan-cancer). In normal tissues, STAMP was hypomethylated, while in tumor tissues, it was hypermethylated. It can be used for clinical tumor detection and as the basis of designing tumor diagnostic agents.

### Term

As used herein, "isolated" refers to a material separated from its original environment (if the material is a natural material, the original environment is the natural environment). For example, in living cells, polynucleotides and polypeptides in their natural state are not isolated or purified, but the same polynucleotides or polypeptides will be isolated ones if they are separated from other substances existed in the natural state.

As used herein, "sample" includes substances suitable for DNA methylation detection obtained from any individual or isolated tissue, cell or body fluid (such as plasma).

As used herein, "hypermethylation" refers to high level of methylation, hydroxymethylation, formylcytosine or carboxylcytosine of CpG in a gene sequence. For example, in the case of methylation specific PCR (MSP), if the PCR reaction with methylation specific primers has positive PCR results, the DNA (gene) region of interest is in hypermethylation state. For another example, in the case of real-time quantitative methylation specific PCR, hypermethylation can be determined based on statistic difference of the methylation status value as compared with the control sample.

As used herein, the tumors include but are not limited to: digestive system tumors such as esophageal cancer (cancer of the esophagus), gastric cancer, colorectal cancer, liver cancer, pancreatic cancer, bile duct and gallbladder cancer; gynecological and reproductive system tumors such as breast cancer, ovarian cancer, cervical cancer, vulvar cancer, testicular cancer, prostate cancer, penile cancer; hematologic cancers such as leukemia, lymphoma, multiple myeloma; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

### Gene marker

In order to find a useful target for tumor diagnosis, the inventor has identified the target of STAMP-EP4 after extensive and in-depth research. The methylation status of the sequence of STAMP-EP4 gene is significantly different between tumor tissues and non-tumor tissues. If abnormal methylation (hypermethylation) is detected in the promoter region of one of the above genes, the subject can be identified as having a high-risk of tumor. Moreover, the significant difference of STAMP-EP4 between tumor and non-tumor tissues exists in various types of tumors, including solid tumors and non-solid tumors.

Therefore, the disclosure provides an isolated polynucleotide of the human genome, comprising the nucleotide sequence shown in the sequence of SEQ ID NO: 1, 2, 5 (the reverse complementary sequence of SEQ ID NO: 1), or 6 (the reverse complementary sequence of SEQ ID NO: 2). For tumor cells, the polynucleotide contains 5-methylcytosine (5mC) at C positions of many 5'-CpG-3'. The disclosure also comprises a fragment of the polynucleotide with a nucleotide sequence as shown in SEQ ID NO: 1, 2, 5 or 6, having at least one (such as 2-54 or 2-204, more specifically 3, 5, 10, 15, 20, 25, 30, 35, 40, 45, 50, 60, 80, 100, 120, 140, 160, 180, 200) methylated CpG site. The above polynucleotides or fragments can also be used in the design of detection agents or detection kits.

In some specific embodiments of the disclosure, the fragment of the polynucleotide are, for example, a fragment containing residues 132 to 164 of SEQ ID NO: 2 (including CpG sites 010-015 of SEQ ID NO: 2); a fragment containing residue 1 to residue 500-529 of SEQ ID NO: 2 (including CpG sites 1-44 of SEQ ID NO: 2); a fragment containing residue 501-530 to residue 1213-1228 of SEQ ID NO: 2 (including CpG sites 45-125 of SEQ ID NO: 2); a fragment containing residue 1214-1229 to residue 1848 of SEQ ID NO: 2 (including CpG sites 126-204 of SEQ ID NO: 2). Antisense strands of the above fragments are suitable for use in the disclosure. Meanwhile, these fragments are merely examples of preferable embodiments of the present disclosure. Based on the information provided by the present disclosure, other fragments can also be selected.

In addition, gene Panels or gene groups containing the sequence shown in the SEQ ID NO: 1, 2, 5 or 6, or a fragment thereof is also encompassed by the disclosure. For the gene Panel or gene group, the characteristics of normal cells and tumor cells can also be identified through DNA methylation detection.

The above polynucleotides can be used as the key regions for analysis of the methylation status in the genome. Their methylation status can be analyzed by various technologies known in the art. Any technique that can be used to analyze the methylation state can be applied to the present disclosure.

When treated with bisulfite, un-methylated cytosine(s) of the above polynucleotides will be converted into uracil, while methylated cytosine(s) remained unchanged.

Therefore, the disclosure also provides the polynucleotides obtained from the above polynucleotides (including the complementary chain (antisense chain)) after being treated with bisulfite, including the polynucleotides with a nucleotide sequence as shown in SEQ ID NO: 3, 4, 7 or 8. These polynucleotides can also be used in the design of detection agents or detection kits.

The disclosure also comprises fragments of the polynucleotides obtained from the above polynucleotides or the antisense strand thereof after being treated with bisulfite, wherein the fragments contain at least one (such as 2-54 or 2-204, more specifically 3, 5, 10, 15, 20, 25, 30, 35, 40, 45, 50, 60, 80, 100, 120, 140, 160, 180, 200) methylated CpG site. The No. of each CpG site in the antisense strand corresponding to the number of the sense strand is readily obtained according to the content described by the present disclosure.

### Detection agents and kits

Based on the new discovery of the disclosure, a detection agent designed based on said polynucleotide(s) is also provided for detecting the methylation profile of polynucleotide(s) in the sample *in vitro.* The detection methods and agents known in the art for determining the sequence and methylation of the genome can be applied in the disclosure.

Therefore, the disclosure provides a method for preparing a tumor detection agent, including: providing the polynucleotide, designing a detection agent for specifically detecting a target sequence which is the full length or fragment of the polynucleotide; wherein, the target sequence has at least one methylated CpG site.

The detection agent herein includes but is not limited to: primers or probes, etc.

For example, the agent is primer pairs. Based on the sequence of the polynucleotide, those skilled in the art know how to design primer(s). The two primers are on each side of the specific sequence of the target gene to be amplified (including CpG sequence, for the gene region originally methylated, the primer is complementary with CpG, and for the gene region originally un-methylated, the primer is complementary with TpG). It should be understood that based on the new discovery of the disclosure, those skilled in the art can design a variety of primers or probes or other types of detection agents for CpG sites at different positions on the target sequence or their combinations. These primers or probes or other types of detection agents should be included in the technical solution of the disclosure. In preferable embodiment of the present disclosure, the primers are selected from the group consisting of: the primers shown in SEQ ID NO: 9 and 10; the primers shown in SEQ ID NO: 11 and 12; the primers shown in SEQ ID NO: 13 and 14; the primers shown in SEQ ID NO: 15 and 16; or the primers shown in SEQ ID NO: 17 and 18.

The agent can also be a combination of agents (primer combination), including more than one set of primers, so that the multiple polynucleotides can be amplified respectively.

The disclosure also provides a kit for detecting the methylation profile of polynucleotide in a sample *in vitro,* which comprises container(s) and the above primer pair(s) in the container(s).

In addition, the kit can also include various reagents required for DNA extraction, DNA purification, PCR amplification, etc.

In addition, the kit can also include an instruction for use, which indicates operation steps of the detection and a result judgment standard, for the application of those skilled in the art.

### Detection method

The methylation profile of a polynucleotide can be determined by any technique in the art (such as methylation specific PCR (MSP) or real-time quantitative methylation specific PCR, Methylight), or other techniques that are still developing and will be developed.

Quantitative methylation specific PCR (QMSP) can also be used to detect methylation level. It is a continuous optical monitoring method based on fluorescent PCR, which is more sensitive than MSP. It has high throughput and avoids electrophoresis based result analysis. Other available technologies include conventional methods in the art such as pyrosequencing, bisulfite converstion sequencing, qPCR, second generation sequencing, whole genome methylation sequencing, DNA enrichment detection, simplified bisulfite sequencing or HPLC, and combined gene group detection. It should be understood that, on the basis of the new disclosure herein, these well-known technologies and some technologies to be developed in the art can be applied to the present disclosure.

As a preferable embodiment of the disclosure, a method of detecting the methylation profile of a polynucleotide in a sample *in vitro* is also provided. The method is based on the follow principle: the un-methylated cytosine can be converted into uracil by bisulfite, which can be transformed into thymine in the subsequent PCR amplification process, while the methylated cytosine remains unchanged; therefore, after the polynucleotide is treated by bisulfite, the methylated site presents a polynucleotide polymorphism (SNP) similar to C/T. Based on the above principle, methylated and un-methylated cytosine can be distinguished effectively by identifying the methylation profile of a polynucleotide in the sample.

The method of the disclosure includes: (a) providing samples and extracting genomic DNA; (b) treating the genomic DNA of step (a) with bisulfite, so as to convert the un-methylated cytosine in the genomic DNA into uracil; (c) analyzing whether the genomic DNA treated in step (b) contains an abnormal methylation profile.

The method of the disclosure can be used for: (i) analyzing whether a subject has tumor by detecting the sample of the subject; (ii) identifying a population having high-risk of tumor. The method needs not to be aimed at obtaining direct diagnosis results.

In a preferable embodiment of the disclosure, DNA methylation is detected by PCR amplification and pyrosequencing. It should be understood by those in the art that DNA methylation detection is not limited to these methods, and any other DNA methylation detection method can be used. The primers used in PCR amplification are not limited to those provided in Examples.

Because of bisulfite treatment, in which un-methylated cytosine in genomic DNA are converted into uracil and then transformed into thymine in the subsequent PCR process, the sequence complexity of the genome will be reduced, and it will be more difficult to amplify specific target fragments by PCR. Therefore, in order to improve amplification efficiency and specificity, nested PCR may be preferable, wherein two sets of primers (outer primers and inner primers) are used in two successive runs of PCR, and the amplification product from the first run undergoes a second run with the second set of primers. However, it should be understood that the detection methods suitable for the present disclosure are not limited thereto.

After the research and verification on clinical samples, the method of the disclosure provides very high accuracy in the clinical diagnosis of tumors. The disclosure can be applied to the fields of tumor auxiliary diagnosis, efficacy evaluation, prognosis monitoring, etc., thus has a high clinical value.

The disclosure is further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Example 1. Nucleic acid sequence for STAMP-EP4 detection

The sequence 1 of the STAMP-EP4 tumor marker is provided as follows: SEQ ID NO: 1 (chr6:391206-391694/hg19), in which the underlined bases are methylated CpG sites, and each number below the underline indicates the site number.

CGCGGAGAGGCAGGGTTCCCGGTGATGGCCTTGCCGAGGGTGCTCCCGCAACCTCC
01 02 03 04 05

ACCTCCAGTTCTCTTTGGACCATTCCTCCGTCTTCCGTTACACGCTCTGCAAAGCGAA
06 07 08 09

GTCCCCTTCGCACCAGATTCCCGCTACTACACGCCCCCCATTTCCCGCCCTGGCCAC
10 11 12 13

ATCGCTGCAGTTTAGTGATTGACTGGCCTCCTGAGGTCCTGGCGCAAAGGCGAGATT
14 15 16

CGCATTTCGCACCTCGCCCTTCGCGGGAAACGGCCCCAGTGACAGTCCCCGAAGCG
17 18 19 20 21 22 23 24

GCGCGCGCCCGGCTGGAGGTGCGCTCTCCGGGCGCGGCGCGCGGAGGGTCGCCAAG
25 26 27 28 29 30 31 32 33 34 35 36

GGCGCGGGAACCCCACCCCGGCCGCGGCAGCCCCCAGCCTTCACGCCGGCCCTGAG
37 38 39 40 41 42 43

GCTCGCCCGCCCGGCCGGCCCCGGCTCTCGGCTTGCAAAGTCCCTCTCCCCAGTCCA
44 45 46 47 48 49

ACCCCCGGCCCCCACAGGCCTCGGCGCCCCGCCCCG
50 51 52 53 54

The sequence 2 of the STAMP-EP4 tumor marker is provided as follows: SEQ ID NO: 2 (chr6:391780-393627/hg19), in which the underlined bases are methylated CpG sites, and each number below the underline indicates the site number.

CGCTGCCCTCAGCTCCGAGTCCAGGGCGAGGTAAGGGCTGGAGTCGGGCAGGAGG
01 02 03 04

AGGGGTGTGAGGCTGATACCAGAGAGGACCCGGAGCGCGAACCAGAGGTTCGACC
05 06 07 08

TCCAGGGCAGCGCAGGGTACCCCGGCTTCGGAGCGGGAAGGGAGCGCGCCCCGTC
09 10 11 12 13 14 15

CTGGAGCTCCGACTCCCACCCCATCTGCGCTGAGCCGGAGGCGCTGGTTTGGGCTC
16 17 18 19

CAAGGCCCGCCTCCTTGGCTCTGCCCGAGCCTCCCCGCCTGCCCTCCGCGCTCCTG
20 21 22 23 24

CGACGGGGTCGCCACAAGCTGGACGGGATGAGCTAACCGGACTGTCGGGCCCCA
25 26 27 28 29 30

GGAGTGGCTGAGGCGGGGCCGTCCAAGGCACCCACACAAGACGGCACAACTGCCT
31 32 33

GCGAGAAACAGGCCCGGCCCTGTGGACCCCAATCCGAGGCTCCTTCCCCTGCTCTT
34 35 36

CGTTCCTAAGGGGCCCAAGCTCACGGCGGCCTCCGGCGCGGTGCTCACCCGCTGG
37 38 39 40 41 42 43

CGCAGGAGGAGGAGCTCCACATTTGGGTCGCTCCGAGCCTTGCGTGCGGTGG
44 45 46 47 48

CCTAGCCGGCCTGGCGCGGTCCCTGCCTCCCAGGCTCCGCAGCTGTCGTCCCCCTCT
49 50 51 52 53 54

CCCGCGCCCTCCCCGCCTCCGCTCTCCCGGGCCTGCTCCGGGGTCCGGCGGACGCT
55 56 57 58 59 60 61 62 63

CTGCGCGCGGAATCCCCCGTACTGGGGCTGCAGCCCCCGCGTCTGCGCCACTTGT
64 65 66 67 68 69 70

CGTTTGCAGAGCCCACTTAGTGCGCGCTAGCTGGGCAGGGATAGGGGTCCTATTCGG
71 72 73 74

GGCCGAAGGGTCTGGATGCGAGCAGAGAAAGCGGAGGGTGGAGGAACCCGGGGCT
75 76 77 78

GCGCCCCTGGAACGCCCGGCCGCAGGCGAGGTCCTCCGCGCGTGGAGGCCGCCAG
79 80 81 82 83 84 85 86 87

GGGAGTGGAAACTGACAGAGTCGCGGGGAAGGGGCGAGAAGCGGGTTGGGAGTG
88 89 90 91
AGCGAAGGCAAGCGAGAGCTGCGAGTGAGTGCGGAAGGAGGGCCAGGAGGGGTG
92 93 94 95
GCGGCTGGGTGGGGAGAGAGGGTGCAAGACGAGCGGCGCGTGTCGGGAGCCTTTG
96 97 98 99 100 101
GGCTGCGGGTGCGTTACAGGAGAGCAGGCGGGTAGGAGCCTTCGCGGGGGCCGAG
102 103 104 105 106 107
CTCGGAAGGCGGACGGCTGTGCCCGCCCAGGGGATGCGCCCGGGCCGGCCGCGAA
108 109 110 111 112 113 114 115 116
GGTGCCTTCTTCCGGGGGCCCGGACGACCCTGACACGGCACGCGCGCGCTTCGCAG
117 118 119 120 121 122 123 124 125
CCTCAAAGACTCCGGGGCCTCGTGGTCACTGGCGCAGGGGATCGGGGCGGGGTGC
126 127 128 129 130
CCGGAGTGCGGTGCCTCGTGGCTGAAGGGCAGCTCTTCTCCCCGCAGTGCAGAGCA
131 132 133 134
GAGCGGGCGGAGGACCCCGGGCGCGGGCGCGGACGGCACGCGGGGCATGAACCT
135 136 137 138 139 140 141 142 143 144
GGAGGCCGGCGGCCGAGGCGGAGAGTTCGGCATGAGCGCGGTGAGCTGCGGCAA
145 146 147 148 149 150 151 152
CGGGAAGCTCCGCCAGTGGCTGATCGACCAGATCGACAGCGGCAAGTACCCCGGG
153 154 155 156 157 158
CTGGTGTGGGAGAACGAGGAGAAGAGCATCTTCCGCATCCCCTGGAAGCACGCGG
159 160 161 162
GCAAGCAGGACTACAACCGCGAGGAGGACGCCGCGCTCTTCAAGGTCTCCGGCCT
163 164 165 166 167 168
CGGGAGCCGGCGGGGGCGCGCCGGGGAGGGCCCAGAGACAGAGCCCGGGGTCCC
169 170 171 172 173 174 175
CGGCGCCGCCTCCGAGGCGAGCCCAGGGGACCGCGCGGGGCGGACGGGCGGGCG
176 177 178 179 180 181 182 183 184 185 186 187
GCGGAGGCATCAGGTGGCGTCGCCGGAGCCGCAGGAGGAGGAAAGGAGGCCTCG
188 189 190 191 192 193
GCTCTCAGCGGGACCGCGGGGGCCGGGAGCCGGGTCCTGGGCGCGTGGAGGCTGC
194 195 196 197 198 199 200
AGGGAAACCGCTGAAGGCCCGGCCGGGCCCG
201 202 203 204

The bisulfite treated sequence of SEQ ID NO: 1 is shown in SEQ ID NO: 3 (Y represents C or U) as follows:

YGYGGAGAGGUAGGGTTUUYGGTGATGGUUTTGUYGAGGGTGUTUUYGUAAUUTU
01 02 03 04 05
UAUUTUUAGTTUTUTTGGAUUATTUUTUYGTUTTUYGTTAUAYGUTUTGUAAAGYG
06 07 08 09
AAGTUUUUTTYGUAUUAGATTUUYGUTAUTAUAYGUUUUUUATTTUUYGUUUTG
10 11 12 13
GUUAUATYGUTGUAGTTTAGTGATTGAUTGGUUTUUTGAGGTUUTGGYGUAAAGGYGA
14 15 16
GATTYGUATTTYGUAUUTYGUUUTTYGYGGGAAAYGGUUUUAGTGAUAGTUUUYGAAGYG
17 18 19 20 21 22 23 24
GYGYGYGUUYGGUTGGAGGTGYGUTUTUYGGGYGYGGYGYGYGGAGGGTYGUUA
25 26 27 28 29 30 31 32 33 34 35 36
AGGGYGYGGGAAUUUUAUUUYGGUYGYGGUAGUUUUAGUUTTUAYGUYGGU
37 38 39 40 41 42 43
UUTGAGGUTYGUUYGUUYGGUYGGUUUYGGUTUTYGGUTTGUAAAGTUUUTUTU
44 45 46 47 48 49
UUUAGTUUAAUUUUYGGUUUUUAUAGGUUTYGGYGUUUYGUUUYG
50 51 52 53 54

The bisulfite treated sequence of SEQ ID NO: 2 is shown in SEQ ID NO: 4 (Y represents C or U) as follows:

YGUTGUUUTUAGUTUYGAGTUUAGGGYGAGGTAAGGGUTGGAGTYGGGUAGGAGGAGG
GGTGTGAGGUTGATAUUAGAGAGGAUUYGGAGYGYGAAUUAGAGGTTYGAUUTUUAGG
GUAGYGUAGGGTAUUUYGGUTTYGGAGYGGGAAGGGAGYGYGUUUYGTUUTGGAGUTUY
GAUTUUUAUUUUAUTUGYGUTGAGUYGGAGGYGUTGGTTTGGGUTUUAAGGUUYGUUT
UUTTGGUTUTGUUYGAGUUTUUUYGUUTGUUTUYGYGUTUUTGYGAYGGGGTYGUUA
UAAGUTGGAYGGGATGAGUTAAUYGGAUTGTYGGGGUUUUAGGAGTGGUTGAGGYGGG
GUYGTUUAAGGUAUUUAUAUAAGAYGGUAUAAUTGUUTGYGAGAAAUAGGUUYGGUU
UTGTGGAUUUUAATUYGAGGUTUUTTUUUUTGUTUTTYGTTUUTAAGGGGUUUAAGUTU
AYGGYGGUUTUYGGYGYGGTGTGUTUAUUYGUTGGYGUAGGAGGAGGAGGAGUTUUAUAT
TTGGGTYGUTUYGAGUUTGYGTGYGGTGGUUTAGUYGGUUTGGYGYGGTUUUTGUUTU
UUAGGUTUYGUAGUTGTYGTYGUUUTUTUUYGYGUUUTUUUYGUUTUYGUTUTUUYGG
GUUTGUTUYGGGGTUYGGYGGAYGUTUTGYGYGYGGAATUUUUYGTAUTGGGGUTGUA
GUUUUYGYGTUTGYGUUAUTTGTYGTTTGUAGAGUUUAUTTAGTGYGYGUTAGUTGGGU
AGGGATAGGGGTUUTATTYGGGGYGAAGGGUTUGGATGYGAGUAGAGAAAGYGGAGGG
TGGAGGAAUUYGGGGUTGYGUUUUTGGAAYGUUYGGUYGUAGGYGAGGTUUTUYGYG
YGTGGAGGUYGUUAGGGGAGTGGAAAUTGAUAGAGTYGYGGGGAAGGGGYGAGAAGY
GGGTTGGGAGTGAGYGAAGGUAAGYGAGAGUTGYGAGTGAGTGYGGAAGGAGGGUUAG
GAGGGGTGGYGGUTGGGTGGGGAGAGAGGGTGUAAGAYGAGYGGYGYGTGTYGGGAGU
UTTTGGGUTGYGGGTGYGTTAUAGGAGAGUAGGYGGGTAGGAGUUTTYGYGGGGGUYG
AGUTYGGAAGGYGGAYGGUTGTGUUYGUUUAGGGGATGYGUUYGGGUYGGUYGYGAA
GGTGUUTTUTTUYGGGGGUUYGGAYGAUUTGAUAYGGUAYGYGYGYGUTTYGUAGUU
TUAAAGAUTUYGGGGUUTYGTGGUUAUTGGYGUAGGGGATYGGGGYGGGGTGUUYGGA
GTGYGGTGUUTYGTGGUTGAAGGGUAGUTUUTUTUUUYGUAGTGUAGAGUAGAGYGGG
YGGAGGAUUUYGGGYGYGGGYGYGGAYGGUAYGYGGGGUATGAAUUTGGAGGGYGGY
GGUYGAGGYGGAGAGTTYGGUATGAGYGYGGTGAGUTGYGGUAAYGGGAAGUTUYGU
UAGTGGUTGATYGAUUAGATYGAUAGYGGUAAGTAUUYGGGUTGGTGTGGGAGAAYG
AGGAGAAGAGUATUTTUYGUATUUUTGGAAGUAYGYGGGUAAGUAGGAUTAUAAUYG
YGAGGAGGAYGUYGYGUTUTTUAAGGTUTUYGGUUTYGGGAGUYGGYGGGGGYGYGU
YGGGGAGGGUUUAGAGAUAGAGUUYGGGGTUUUYGGYGUYGUUTUYGAGGYGAGUUU
AGGGGAUYGYGYGGGGYGGAYGGGYGGGYGGYGGAGGUATUAGGTGGYGTYGUYGGA
GUYGUAGGAGGAGGAAAGGAGGUUTYGGUTUTUAGYGGGAUYGYGGGGGUYGGGAGU
YGGGTUUTGGGYGYGTGGAGGUTGUAGGGAAAUYGUTGAAGGUUYGGUYGGGUUYG

The reverse complementary sequence of SEQ ID NO: 1 is shown in SEQ ID NO: 5 as follows:

CGGGCGGGCGCCGAGGCCTGTGGGGCCGGGGTTGGACTGGGAGAGGGACTTTGC
AAGCCGAGAGCCGGGGCCGGCCGGGCGGGCGAGCCTCAGGGCCGGCGTGAAGGCTGGGG
GCTGCCGCGGCCGGGGTGGGGTTCCCGCGCCCTTGGCGACCCTCCGCGCGCCGCGCCCGG
AGAGCGCACCTCCAGCCGGGCGCGCGCCGCTTCGGGGACTGTCACTGGGGCCGTTTCCCGC
GAAGGGCGAGGTGCGAAATGCGAATCTCGCCTTTGCGCCAGGACCTCAGGAGGCCAGTCA
ATCACTAAACTGCAGCGATGTGGCCAGGGCGGGAAATGGGGGGCGTGTAGTAGCGGGAAT
CTGGTGCGAAGGGGACTTCGCTTTGCAGAGCGTGTAACGGAAGACGGAGGAATGGTCCAA
AGAGAACTGGAGGTGGAGGTTGCGGGAGCACCCTCGGCAAGGCCATCACCGGGAACCCTG
CCTCTCCGCG

The reverse complementary sequence of SEQ ID NO: 2 is shown in SEQ ID NO: 6 as follows:

CGGGCCCGGCCGGGCCTTCAGCGGTTTCCTGCAGCCTCCACGCGCCCAGGACCCGGCTC
CCGGCCCCCGCGGTCCCGCTGAGAGCCGAGGCCTCCTTTCCTCCTCCTGCGGCTCCGGCG
ACGCCACCTGATGCCTCCGCCGCCCGCCCGTCCGCCCCGCGCGGTCCCCTGGGCTCGCCT
CGGAGGCGGCGCCGGGGACCCCGGGCTCTGTCTCTGGGCCCTCCCCGGCGCGCCCCCGC
CGGCTCCCGAGGCCGGAGACCTTGAAGAGCGCGGCGTCCTCCTCGCGGTTGTAGTCCTGC
TTGCCCGCGTGCTTCCAGGGGATGCGGAAGATGCTCTTCTCCTCGTTCTCCCACACCAGCC
CGGGGTACTTGCCGCTGTCGATCTGGTCGATCAGCCACTGGCGGAGCTTCCCGTTGCCGC
AGCTCACCGCGCTCATGCCGAACTCTCCGCCTCGGCCGCCGCCCTCCAGGTTCATGCCCC
GCGTGCCGTCCGCGCCCGCGCCCGGGGTCCTCCGCCCGCTCTGCTCTGCACTGCGGGGAG
AAGAGCTGCCCTTCAGCCACGAGGCACCGCACTCCGGGCACCCCGCCCCGATCCCCTGCG
CCAGTGACCACGAGGCCCCGGAGTCTTTGAGGCTGCGAAGCGCGCGCGTGCCGTGTCAGG
GTCGTCCGGGCCCCCGGAAGAAGGCACCTTCGCGGCCGGCCCGGGCGCATCCCCTGGGC
GGGCACAGCCGTCCGCCTTCCGAGCTCGGCCCCCGCGAAGGCTCCTACCCGCCTGCTCTC
CTGTAACGCACCCGCAGCCCAAAGGCTCCCGACACGCGCCGCTCGTCTTGCACCCTCTCT
CCCCACCCAGCCGCCACCCCTCCTGGCCCTCCTTCCGCACTCACTCGCAGCTCTCGCTTGC
CTTCGCTCACTCCCAACCCGCTTCTCGCCCCTTCCCCGCGACTCTGTCAGTTTCCACTCCCC
TGGCGGCCTCCACGCGCGGAGGACCTCGCCTGCGGCCGGGCGTTCCAGGGGCGCAGCCC
CGGGTTCCTCCACCCTCCGCTTTCTCTGCTCGCATCCAGACCCTTCGCCCCGAATAGGACC
CCTATCCCTGCCCAGCTAGCGCGCACTAAGTGGGCTCTGCAAACGACAAGTGGCGCAGAC
GCGGGGGCTGCAGCCCCAGTACGGGGGATTCCGCGCGCAGAGCGTCCGCCGGACCCCGG
AGCAGGCCCGGGAGAGCGGAGGCGGGGAGGGCGCGGGAGAGGGCGACGACAGCTGCGG
AGCCTGGGAGGCAGGGACCGCGCCAGGCCGGCTAGGCCACCGCACGCAAGGCTCGGAGC
GACCCAAATGTGGAGCTCCTCCTCCTCCTGCGCCAGCGGGTGAGCACCGCGCCGGAGGCC
GCCGTGAGCTTGGGCCCCTTAGGAACGAAGAGCAGGGGAAGGAGCCTCGGATTGGGGTC
CACAGGGCCGGGCCTGTTTCTCGCAGGCAGTTGTGCCGTCTTGTGTGGGTGCCTTGGACG

GCCCCGCCTCAGCCACTCCTGGGGCCCCGACAGTCCGGTTAGCTCATCCCGTCCAGCTTGT
GGCGACCCCGTCGCAGGAGCGCGGAGGGCAGGCGGGGAGGCTCGGGCAGAGCCAAGGA
GGCGGGCCTTGGAGCCCAAACCAGCGCCTCCGGCTCAGCGCAGATGGGGTGGGAGTCGG
AGCTCCAGGACGGGGCGCGCTCCCTTCCCGCTCCGAAGCCGGGGTACCCTGCGCTGCCCT
GGAGGTCGAACCTCTGGTTCGCGCTCCGGGTCCTCTCTGGTATCAGCCTCACACCCCTCCT
CCTGCCCGACTCCAGCCCTTACCTCGCCCTGGACTCGGAGCTGAGGGCAGCG

The bisulfite treated sequence of SEQ ID NO: 5 is shown in SEQ ID NO: 7 (Y represents C or U) as follows:

YGGGGYGGGGYGUYGAGGUUTGTGGGGGUYGGGGGTTGGAUTGGGAGAGGGAUTTTGU
AAGUYGAGAGUYGGGGUYGGUYGGGYGGGYGAGUUTUAGGGUYGGYGTGAAGGUTGGG
GGUTGUYGYGGUYGGGGTGGGGTTUUYGYGUUUTTGGYGAUUUTUYGYGYGUYGYGUU
YGGAGAGYGUAUUTUUAGUYGGGYGYGYGUYGUTTYGGGGAUTGUAUTGGGGUYGTTT
UUYGYGAAGGGYGAGGTGYGAAATGYGAATUTYGUUTTGYGUUAGGAUUTUAGGAGGU
UAGTUAATUAUTAAAUTGUAGYGATGTGGUUAGGGYGGGAAATGGGGGGYGTGTAGTAG
YGGGAATUTGGTGYGAAGGGGAUTTYGUTTTGUAGAGYGTGTAAYGGAAGAYGGAGGAA
TGGTUUAAAGAGAAUTGGAGGTGGAGGTTGYGGGAGUAUUUTYGGUAAGGUUATUAUYG
GGAAUUUTGUUTUTUYGYG

The bisulfite treated sequence of SEQ ID NO: 6 is shown in SEQ ID NO: 8 (Y represents C or U) as follows:

YGGGUUYGGUYGGGUUTTUAGYGGTTTUUUTGUAGUUTUUAYGYGUUUAGGAUUYGGU
TUUYGGUUUUYGYGGTUUYGUTGAGAGUYGAGGUUTUUTTUUTUUTUUTGYGGUTUY
GGYGAYGUUAUUTGATGUUTUYGUYGUUYGUUYGTUYGUUUYGYGYGGTUUUUTGGG
UTYGUUTYGGAGGYGGYGUYGGGGAUUUYGGGUTUTGTUTUTGGGUUUTUUUYGGYGY
GUUUUYGUYGGUTUUYGAGGUYGGAGAUUTTGAAGAGYGYGGYGTUUTUUTYGYGGTT
GTAGTUUTGUTTGUUYGYGTGUTTUUAGGGGATGYGGAAGATGUTUTTUTUUTYGTTUTU
UUAUAUUAGUUYGGGGTAUUTGUYGUTGTYGATUTGGTYGATUAGUUAUTGGYGGAGU
TTUUYGTTGUYGUAGUTUAUYGYGUTUATGUYGAAUTUTUYGUUTYGGUYGUYGUUUT
UUAGGTTUATGUUUYGYGTGUYGTUYGYGUUYGYGUUYGGGGTUUTUYGUUYGUTUTG
UTUTGUAUTGYGGGGAGAAGAGUTGUUUTTUAGUUAYGAGGUAUYGUAUTUYGGGUAU
UUYGUUUYGATUUUUTGYGUUAGTGAUUAYGAGGUUUYGGAGTUTTGAGGUTGYGAA
GYGYGYGYGTGUYGTGTUAGGGTYGTUYGGGUUUUYGGAAGAAGGUAUUTTYGYGGUY
GGUUYGGGYGUATUUUUTGGGYGGGUAUAGUYGTUYGUUTUYGAGUTYGGUUUUYG
YGAAGGUTUUTAUUYGUUTGUTUTUUTGTAAYGUAUUYGUAGUUUAAAGGUTUUYGAU
AYGYGUYGUTYGTUTTGUAUUTUTUTUUUUAUUUAGUYGUUAUUUTUUTGGUUUTU
UTTUYGUAUTUAUTYGUAGUTUTYGUTTGUUTTYGUTUAUTUUUAAUUYGUTTUTYGUU
UUTTUUUYGYGAUTUTGTUAGTTTUUAUTUUUTGGYGGUUTUUAYGYGYGGAGGAUUT
YGUUTGYGGUYGGGYGTTUUAGGGGYGUAGUUUYGGGTTUUTUUAUUUTUYGUTTTUT
UTGUTYGUATUUAGAUUUTTYGUUUYGAATAGGAUUUUTATUUTGUUUAGUTAGYGY
GUAUTAAGTGGGUTUTGUAAAYGAUAAGTGGYGUAGAYGYGGGGGUTGUAGUUUAGT
AYGGGGGATTUYGYGYGUAGAGYGTUYGUYGGAUUUYGGAGUAGGUUYGGGAGAGYG
GAGGYGGGGAGGGYGYGGGAGAGGGYGAYGAUAGUTGYGGAGUUTGGGAGGUAGGGA
UYGYGUUAGGUYGGUTAGGUUAUYGUAYGUAAGGUTYGGAGYGAUUUAAATGTGGAG
UTUUTUUTUUTUUTGYGUUAGYGGGTGAGUAUYGYGUYGGAGGUYGUYGTGAGUTTGG
GUUUUTTAGGAAYGAAGAGUAGGGGAAGGAGUUTYGGATTGGGGTUUAUAGGUYGGG
UUTGTTTUTYGUAGGUAGTTGTGUYGTUTTGTGTGGGTGUUTTGGAYGGUUUYGUUTUA
GUUAUTUUTGGGGUUUYGAUAGTUYGGTTAGUTUATUUYGTUUAGUTTGTGGYGAUUU
YGTYGUAGGAGYGYGGAGGGUAGGYGGGGAGGUTYGGGUAGAGUUAAGGAGGYGGGU
UTTGGAGUUUAAAUUAGYGUUTUYGGUTUAGYGUAGATGGGGTGGGAGTYGGAGUTUU
AGGAYGGGGYGYGUTUUUTTUUYGUTUYGAAGUYGGGGTAUUUTGYGUTGUUUTGGAG
GTYGAAUUTUTGGTTYGYGUTUYGGGTUUTUTUTGGTATUAGUUTUAUAUUUTUUTUU
TGUUYGAUTUUAGUUUTTAUUTYGUUUTGGAUTYGGAGUTGAGGGUAGYG

### Example 2. Difference in methylation of STAMP-EP4 CpG sites between tumor cells and non-tumor cells -- Bisulfite Sequencing PCR (BSP)

1. Genomic DNA was extracted from liver cancer cell line HepG2 and normal liver cell line;
2. The extracted genomic DNA of HepG2 and normal liver cell lines were treated with bisulfite and used as templates for subsequent PCR amplification; EZ DNA Methylation-Gold Kit (ZYMO Research, Cat# D5006) was used in this experiment, which is not limited thereto;
3. Primers (SEQ ID NO: 9-6; Table 1) were designed according to SEQ ID NO: 1 or 2, respectively, by conventional methods for amplification of different sequence regions;
4. After PCR amplification, 2% agarose gel electrophoresis was used to detect the specificity of the PCR fragments. The target fragments were recovered by gel cutting and connected to T vector which was transformed into competent *Escherichia coli.* The bacteria were spread on plate, and clones were selected the next day and sequenced. Ten clones were selected for each fragment for Sanger sequencing.

**Table 1, BSP primer**

| Primer Name | 5'-3' Primer Sequence | Detected CpG site No. |
|---|---|---|
| STAMP-EP4-San ger-1-Primer-F | TTAGTGAGTGYGATGTTTTTTAAATAT (SEQ ID NO: 9) | CpG 001-054 of the above SEQ ID NO: 1 |
| STAMP-EP4-San ger-1-Primer-R | ATAAAACTCTCTAAAACRAAACCTAAAAC (SEQ ID NO: 10) | |
| STAMP-EP4-San ger-2-Primer-F | GTATTTTTAGTTTTATYGTTYGATTTTGGGAT(SEQ ID NO: 11) | CpG 001-044 of the above SEQ ID NO: 2 |
| STAMP-EP4-San ger-2-Primer-R | CCAAATATAAAACTCCTCCTCCTCCTAC (SEQ ID NO: 12) | |
| STAMP-EP4-San ger-3-Primer-F | TAGGAGGAGGAGGAGTTTTATATTTGGGT (SEQ ID NO: 13) | CpG 045-125 of the above SEQ ID NO: 2 |
| STAMP-EP4-San ger-3-Primer-R | AACCACRAAACCCCRAAATCTTTAAAACTAC(SEQ ID NO: 14) | |
| STAMP-EP4-San ger-4-Primer-F | GTGTTTTTTTTYGGGGGTTYGGAYGATTTTGATA(SE Q ID NO: 15) | CpG 126-204 of the above SEQ ID NO: 2 |
| STAMP-EP4-San ger-4-Primer-R | AACCCCTCAAACCTTTAACCRCCCTTCCCC(SEQ ID NO: 16) | |

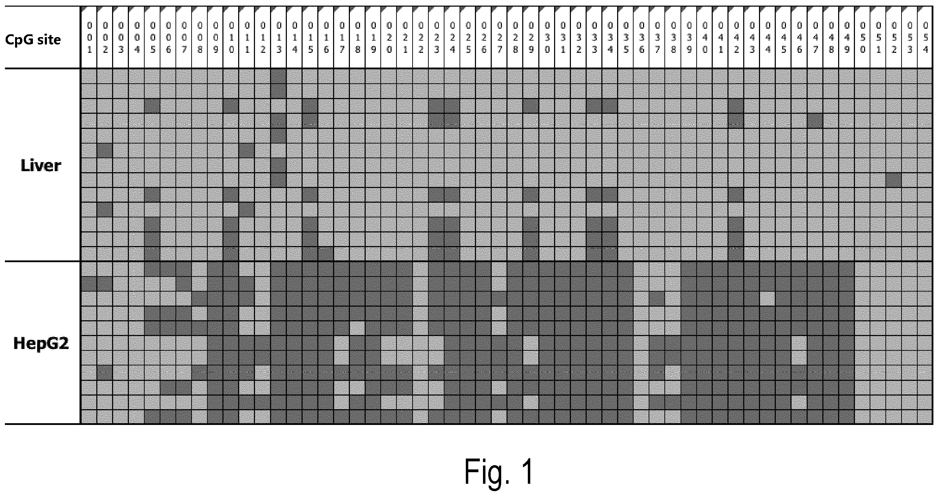

Difference in methylation levels of methylation sites 001-054 of SEQ ID NO:1 in normal liver cells and liver cancer cells is shown in Figure 1.

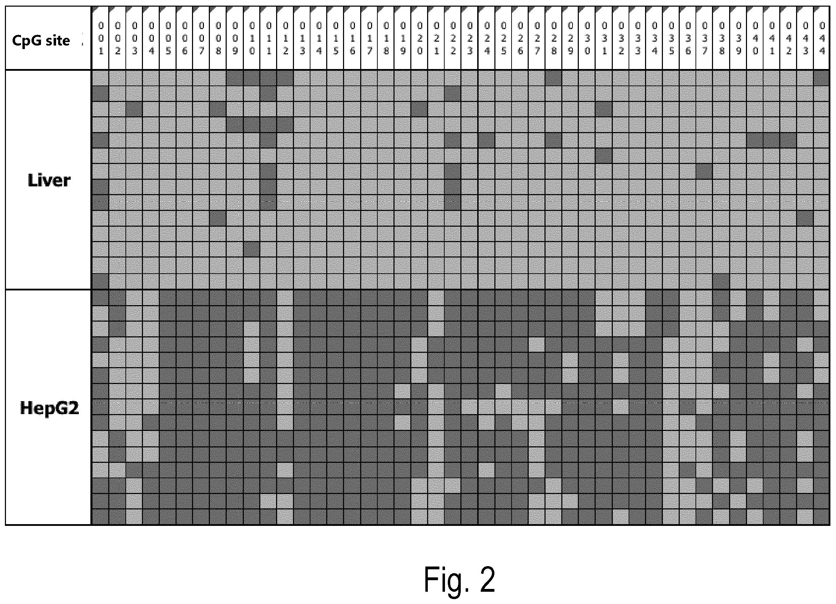

Difference in methylation levels of methylation sites 001-044 of SEQ ID NO:2 in normal liver cells and liver cancer cells is shown in Figure 2.

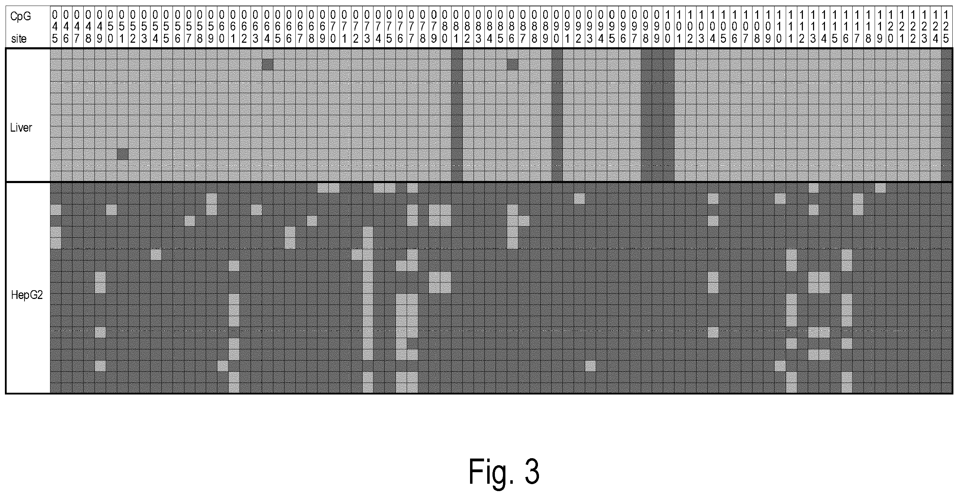

Difference in methylation levels of methylation sites 045-125 of SEQ ID NO:2 in normal liver cells and liver cancer cells is shown in Figure 3.

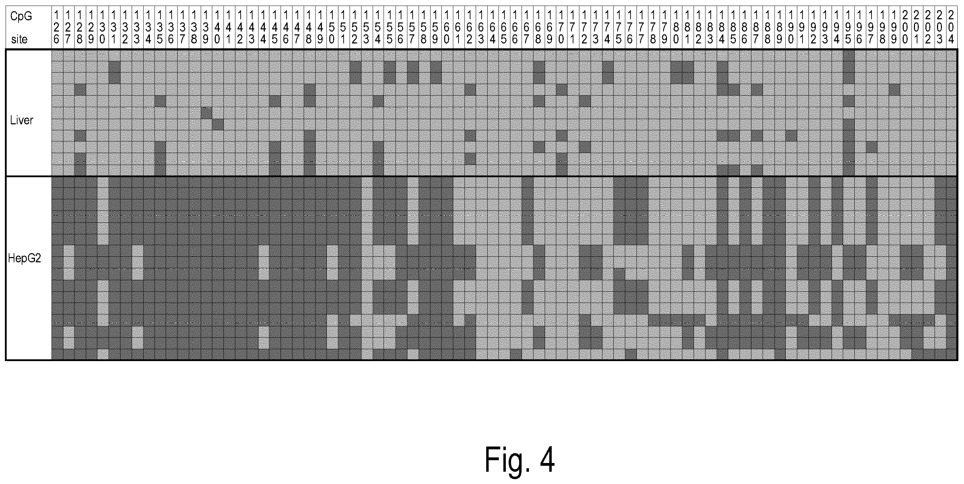

Difference in methylation levels of methylation sites 126-204 of SEQ ID NO:2 in normal liver cells and liver cancer cells is shown in Figure 4.

These results indicate that the methylation levels of regions of SEQ ID NO: 1 and 2 of a liver cancer cell line were significantly higher than that of a normal liver cell line.

### Example 3: Verification of difference in methylation of STAMP-EP4 between tumor and non-tumor clinical samples-pyrosequencing

1. Clinical samples: paracancerous/non-cancer samples were used as the control group, and tumor samples were used as the experimental group;
2. DNA extraction: DNA was extracted from the experimental group and the control group respectively. Phenol-chloroform extraction method was used in this experiment, which is not limited thereto;
3. Bisulfite treatment: the extracted DNA samples were treated with bisulfite and the procedures were strictly followed. EZ DNA Methylation-Gold Kit (ZYMO Research, Cat# D5006) was used in this experiment, which is not limited thereto;
4. Primer design: PCR primers and pyrosequencing primers were designed according to the characteristics of SEQ ID NO: 2 of STAMP-EP4 sequence. The methylation values of STAMP-EP4 were detected as the methylation values of CpG sites 10-15 of SEQ ID NO:2. The PCR primers sequences, pyrosequencing primers sequences, and the pyrosequencing detecting sequences and the detected sites are shown as SEQ ID NO: 17-20 (Table 2);
5. PCR amplification and agarose gel electrophoresis: The bisulfite treated samples were used as templates for PCR amplification. The specificity of PCR amplification was identified by agarose gel electrophoresis of the amplified products;
6. Pyrosequencing: Pyro Mark Q96 ID pyrosequencing instrument (QIAGEN) was used for sequencing, and the procedures in instructions were strictly followed;
7. Calculation of STAMP-EP4 methylation value: pyrosequencing can detect the methylation value of each site in the target region, respectively, and the average methylation value of all sites were calculated as the STAMP-EP4 methylation value in the sample;
8. Results analysis: the methylation value of STAMP-EP4 was compared between the paracancerous/non-cancer control group and the cancer experimental group.

**Table 2, Pyro primer**

| Primer Name | 5'-3' sequence | Remark |
|---|---|---|
| STAMP-EP4-Pyroseq-Primer -F | GATTTTTAGGGTAGYGTAGGGTATTT (SEQ ID NO: 17) | The primer pair detects CpG 010-015 of SEQ |
| STAMP-EP4-Pyroseq-Primer | CAAATAAAATAAAAATCRAAACTCCAAAAC | |

| -R (modified by 5'-Biotin) | (SEQ ID NO: 18) | ID NO: 2 |
|---|---|---|
| STAMP-EP4-Pyroseq-Primer -Seq | GATTTTTAGGGTAGYGTAGGGTATTT (SEQ ID NO: 19) | |
| pyrosequencing detecting sequences | YGGTTTYGGAGYGGGAAGGGAGYGYGTTTY GTTTTGGAGTTTYGA (SEQ ID NO: 20) | |

### Example 4. STAMP-EP4: breast cancer clinical sample assay - pyrosequencing

STAMP-EP4 methylation value between the control group of 5 cases of breast cancer paracancerous clinical samples and the experimental group of 5 cases of breast cancer clinical samples was compared according to the pyrosequencing in Example 3.

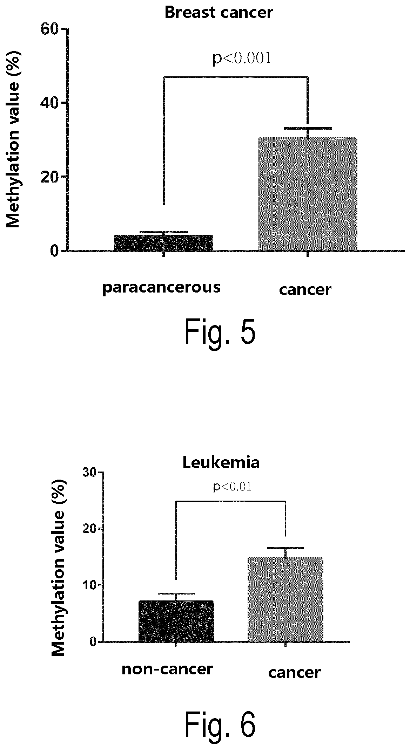

The result in Figure 5 shows that, in clinical samples of breast cancer, the methylation value of STAMP-EP4 in the experimental group was significantly higher than that of paracancerous tissues.

### Example 5. STAMP-EP4: leukemia clinical sample assay - pyrosequencing

STAMP-EP4 methylation value between the control group of 8 leukemia bone marrow smear clinical samples and the experimental group of 8 non-leukemia bone marrow smear clinical samples was compared according to the pyrosequencing in Example 3.

The result in Figure 6 shows that, in clinical samples of leukemia, the methylation value of STAMP-EP4 in the experimental group was significantly higher than that of non-cancer tissues.

### Example 6. STAMP-EP4: colorectal cancer clinical sample assay - pyrosequencing

STAMP-EP4 methylation value was analyzed on the control group of 8 cases of colorectal cancer paracancerous clinical samples and the experimental group of 8 cases of colorectal cancer clinical samples according to the pyrosequencing in Example 3.

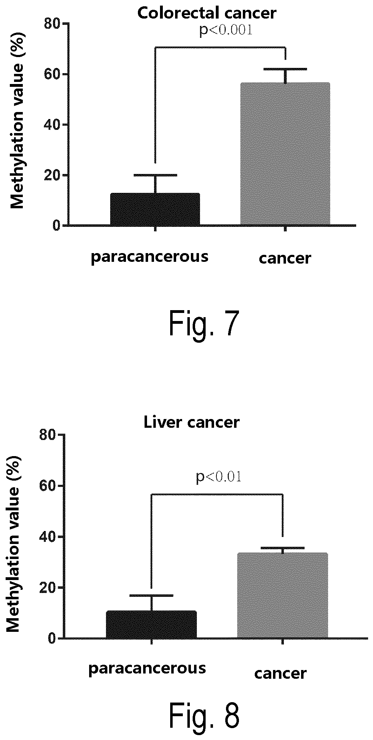

The result in Figure 7 shows that, in clinical samples of colorectal cancer, the methylation value of STAMP-EP4 in the experimental group was significantly higher than that of paracancerous tissues.

### Example 7. STAMP-EP4: liver cancer clinical sample assay - pyrosequencing

STAMP-EP4 methylation value was analyzed on the control group of 8 cases of liver cancer paracancerous clinical samples and the experimental group of 8 cases of liver cancer clinical samples according to the pyrosequencing in Example 3.

The result in Figure 8 shows that, in clinical samples of liver cancer, the methylation value of STAMP-EP4 in the experimental group was significantly higher than that of paracancerous tissues.

### Example 8. STAMP-EP4: lung cancer clinical sample assay - pyrosequencing

STAMP-EP4 methylation value was analyzed on the control group of 4 cases of lung cancer paracancerous clinical samples and the experimental group of 4 cases of lung cancer clinical samples according to the pyrosequencing in Example 3.

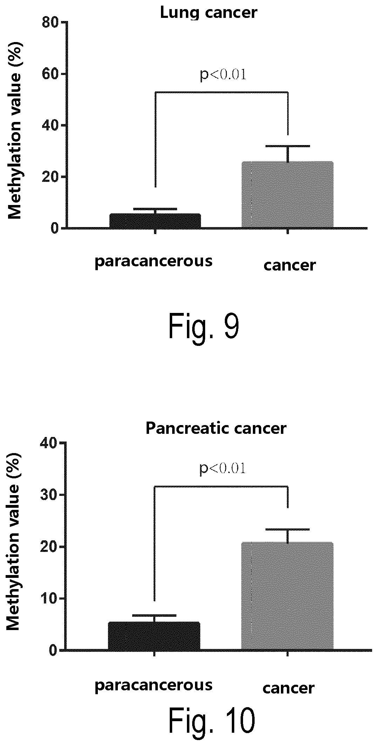

The result in Figure 9 shows that, in clinical samples of lung cancer, the methylation value of STAMP-EP4 in the experimental group was significantly higher than that of paracancerous tissues.

### Example 9. STAMP-EP4: pancreatic cancer clinical sample assay - pyrosequencing

STAMP-EP4 methylation value was analyzed on the control group of 4 cases of pancreatic cancer paracancerous clinical samples and the experimental group of 4 cases of pancreatic cancer clinical samples according to the pyrosequencing in Example 3.

The result in Figure 10 shows that, in clinical samples of pancreatic cancer, the methylation value of STAMP-EP4 in the experimental group was significantly higher than that of paracancerous tissues.

### Example 10. STAMP-EP4: Esophageal cancer clinical sample assay - pyrosequencing

STAMP-EP4 methylation value was analyzed on the control group of 10 cases of esophageal cancer paracancerous clinical samples and the experimental group of 10 cases of esophageal cancer clinical samples according to the pyrosequencing in Example 3.

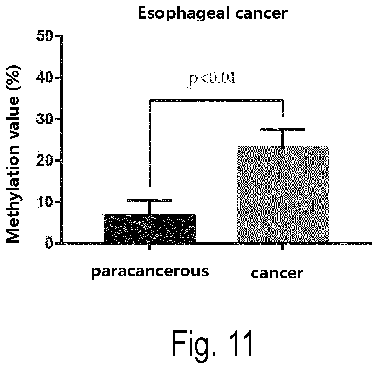

The result in Figure 11 shows that, in clinical samples of esophageal cancer, the methylation value of STAMP-EP4 in the experimental group was significantly higher than that of paracancerous tissues.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

Sequenceing List

<110>  SHANGHAI EPIPROBE BIOTECHNOLOGY CO.,LTD.

<120>  METHYLATION MODIFICATION-BASED TUMOR MARKER STAMP-EP4

<130>  18A293

<160>  20

<170>  SIPOSequenceListing 1.0

<210>  1
<211>  489
<212>  DNA
<213>  Homo sapiens

<400>  1
cgcggagagg cagggttccc ggtgatggcc ttgccgaggg tgctcccgca acctccacct      60
ccagttctct ttggaccatt cctccgtctt ccgttacacg ctctgcaaag cgaagtcccc     120
ttcgcaccag attcccgcta ctacacgccc cccatttccc gccctggcca catcgctgca     180
gtttagtgat tgactggcct cctgaggtcc tggcgcaaag gcgagattcg catttcgcac     240
ctcgcccttc gcgggaaacg gccccagtga cagtccccga agcggcgcgc gcccggctgg     300
aggtgcgctc tccgggcgcg gcgcgcggag ggtcgccaag ggcgcgggaa ccccaccccg     360
gccgcggcag cccccagcct tcacgccggc cctgaggctc gcccgcccgg ccggccccgg     420
ctctcggctt gcaaagtccc tctccccagt ccaacccccg gcccccacag gcctcggcgc     480
cccgccccg                                                             489

<210>  2
<211>  1848
<212>  DNA
<213>  Homo sapiens

<400>  2
cgctgccctc agctccgagt ccagggcgag gtaagggctg gagtcgggca ggaggagggg      60
tgtgaggctg ataccagaga ggacccggag cgcgaaccag aggttcgacc tccagggcag     120
cgcagggtac cccggcttcg gagcgggaag ggagcgcgcc ccgtcctgga gctccgactc     180
ccaccccatc tgcgctgagc cggaggcgct ggtttgggct ccaaggcccg cctccttggc     240
tctgcccgag cctccccgcc tgccctccgc gctcctgcga cggggtcgcc acaagctgga     300
cgggatgagc taaccggact gtcggggccc caggagtggc tgaggcgggg ccgtccaagg     360
cacccacaca agacggcaca actgcctgcg agaaacaggc ccggccctgt ggaccccaat     420
ccgaggctcc ttcccctgct cttcgttcct aaggggccca agctcacggc ggcctccggc     480
gcggtgctca cccgctggcg caggaggagg aggagctcca catttgggtc gctccgagcc     540
ttgcgtgcgg tggcctagcc ggcctggcgc ggtccctgcc tcccaggctc cgcagctgtc     600
gtcgccctct cccgcgccct ccccgcctcc gctctcccgg gcctgctccg gggtccggcg     660
gacgctctgc gcgcggaatc ccccgtactg gggctgcagc ccccgcgtct gcgccacttg     720
tcgtttgcag agcccactta gtgcgcgcta gctgggcagg gataggggtc ctattcgggg     780
cgaagggtct ggatgcgagc agagaaagcg gagggtggag gaacccgggg ctgcgcccct     840
ggaacgcccg gccgcaggcg aggtcctccg cgcgtggagg ccgccagggg agtggaaact     900
gacagagtcg cggggaaggg gcgagaagcg ggttgggagt gagcgaaggc aagcgagagc     960
tgcgagtgag tgcggaagga gggccaggag gggtggcggc tgggtgggga gagagggtgc    1020
aagacgagcg gcgcgtgtcg ggagcctttg ggctgcgggt gcgttacagg agagcaggcg    1080
ggtaggagcc ttcgcggggg ccgagctcgg aaggcggacg gctgtgcccg cccaggggat    1140
gcgcccgggc cggccgcgaa ggtgccttct tccgggggcc cggacgaccc tgacacggca    1200
cgcgcgcgct tcgcagcctc aaagactccg gggcctcgtg gtcactggcg caggggatcg    1260
gggcggggtg cccggagtgc ggtgcctcgt ggctgaaggg cagctcttct ccccgcagtg    1320
cagagcagag cgggcggagg accccgggcg cgggcgcgga cggcacgcgg ggcatgaacc    1380
tggagggcgg cggccgaggc ggagagttcg gcatgagcgc ggtgagctgc ggcaacggga    1440
agctccgcca gtggctgatc gaccagatcg acagcggcaa gtaccccggg ctggtgtggg    1500
agaacgagga gaagagcatc ttccgcatcc cctggaagca cgcgggcaag caggactaca    1560
accgcgagga ggacgccgcg ctcttcaagg tctccggcct cgggagccgg cgggggcgcg    1620
ccggggaggg cccagagaca gagcccgggg tccccggcgc cgcctccgag gcgagcccag    1680
gggaccgcgc ggggcggacg ggcgggcggc ggaggcatca ggtggcgtcg ccggagccgc    1740

aggaggagga aaggaggcct cggctctcag cgggaccgcg ggggccggga gccgggtcct 1800
gggcgcgtgg aggctgcagg gaaaccgctg aaggcccggc cgggcccg 1848

<210> 3
<211> 489
<212> DNA/RNA
<213> Artificial Sequence

<220>
<221> misc_feature
<222> (1)..(489)
<223> bisulfite treated sequence of SEQ ID NO:1


<220>
<221> misc_feature
<222> (1)..(489)
<223> y is C or U


<400> 3
ygyggagagg uagggttuuy ggtgatgguu ttguygaggg tgutuuygua auutuuauut 60
uuagttutut ttggauuatt uutuygtutt uygttauayg ututguaaag ygaagtuuuu 120
ttyguauuag attuuyguta utauayguuu uuuatttuuy guuutgguua uatygutgua 180
gtttagtgat tgautgguut uutgaggtuu tggyguaaag gygagattyg uatttyguau 240
utyguuutty gygggaaayg guuuuagtga uagtuuuyga agyggygygy guuyggutgg 300
aggtgygutu tuygggygyg gygygyggag ggtyguuaag ggygygggaa uuuuauuuyg 360
guygygguag uuuuuaguut tuayguyggu uutgagguty guuyguuygg uygguuuygg 420
ututyggutt guaaagtuuu tutuuuuagt uuaauuuuyg guuuuuauag guutyggygu 480
uuyguuuyg 489

<210> 4
<211> 1848
<212> DNA/RNA
<213> Artificial Sequence

<220>
<221> misc_feature
<222> (1)..(1848)
<223> bisulfite treated sequence of SEQ ID NO:2


<220>
<221> misc_feature
<222> (1)..(1848)
<223> y is C or U


<400> 4
ygutguuutu agutuygagt uuagggygag gtaagggutg gagtygggua ggaggagggg 60
tgtgaggutg atauuagaga ggauuyggag ygygaauuag aggttygauu tuuaggguag 120
yguagggtau uuygguttyg gagygggaag ggagygyguu uygtuutgga gutuygautu 180
uuauuuuatu tgygutgagu yggaggygut ggtttgggut uuaagguuyg uutuuttggu 240
tutguuygag uutuuuyguu tguuutuygy gutuutgyga yggggtyguu auaagutgga 300
ygggatgagu taauyggaut gtygggguuu uaggagtggu tgaggygggg uygtuuaagg 360
uauuuauaua agaygguaua autguutgyg agaaauaggu uygguuutgt ggauuuuaat 420
uygaggutuu ttuuuutgut uttygttuut aagggguuua agutuayggy gguutuyggy 480
gyggtgutua uuygutggyg uaggaggagg aggagtuuua uatttgggty gutuygaguu 540
ttgygtgygg tgguutaguy gguutggygy ggtuuutguu tuuuaggutu yguagutgty 600
gtyguuutut uuygyguuut uuuyguutuy gututuuygg guutgutuyg gggtuyggyg 660
gaygututgy gygyggaatu uuuygtautg gggutguagu uuuygygtut gyguuauttg 720
tygtttguag aguuuautta gtgygyguta gutggguagg gataggggtu utattygggg 780
ygaagggtut ggatgygagu agagaaagyg gagggtggag gaauuygggg utgyguuuut 840

ggaayguuyg guyguaggyg aggtuutuyg ygygtggagg uyguuagggg agtggaaaut 900
gauagagtyg yggggaaggg gygagaagyg ggttgggagt gagygaaggu aagygagagu 960
tgygagtgag tgyggaagga ggguuaggag gggtggyggu tgggtgggga gagagggtgu 1020
aagaygagyg gygygtgtyg ggaguutttg ggutgygggt gygttauagg agaguaggyg 1080
ggtaggaguu ttygyggggg uygagutygg aaggyggayg gutgtguuyg uuuaggggat 1140
gyguuygggu ygguygygaa ggtguuttut tuyggggguu yggaygauuu tgauayggua 1200
ygygygygut tyguaguutu aaagautuyg ggguutygtg gtuautggyg uaggggatyg 1260
gggyggggtg uuyggagtgy ggtguutygt ggutgaaggg uagututtut uuuyguagtg 1320
uagaguagag ygggyggagg auuuygggyg ygggygygga ygguaygygg gguatgaauu 1380
tggagggygg ygguygaggy ggagagttyg guatgagygy ggtgagutgy gguaayggga 1440
agutuyguua gtggutgaty gauuagatyg auagygguaa gtauuuyggg utggtgtggg 1500
agaaygagga gaagaguatu ttuyguatuu uutggaagua ygygggtaag uaggautaua 1560
auygygagga ggayguygyg ututtuaagg tutuygguut ygggaguygg ygggggygyg 1620
uyggggaggg uuuagagaua gaguuygggg tuuuyggygu yguutuygag gygaguuuag 1680
gggauygygy ggggyggayg ggygggyggy ggagguatua ggtggygtyg uyggaguygu 1740
aggaggagga aaggagguut yggututuag ygggauygyg ggggyuygga guygggtuut 1800
gggygygtgg aggutguagg gaaauygutg aagguuyggu ygggguuyg 1848

<210> 5
<211> 489
<212> DNA/RNA
<213> Homo sapiens

<400> 5
cggggcgggg cgccgaggcc tgtgggggcc gggggttgga ctggggagag ggactttgca 60
agccgagagc cggggccggc cgggcgggcg agcctcaggg ccggcgtgaa ggctgggggc 120
tgccgcggcc ggggtggggt tcccgcgccc ttggcgaccc tccgcgcgcc gcgcccggag 180
agcgcacctc cagccgggcg cgcgccgctt cggggactgt cactggggcc gtttcccgcg 240
aagggcgagg tgcgaaatgc gaatctcgcc tttgcgccag gacctcagga ggccagtcaa 300
tcactaaact gcagcgatgt ggccagggcg ggaaatgggg ggcgtgtagt agcgggaatc 360
tggtgcgaag gggacttcgc tttgcagagc gtgtaacgga agacggagga atggtccaaa 420
gagaactgga ggtggaggtt gcgggagcac cctcggcaag gccatcaccg ggaaccctgc 480
ctctccgcg 489

<210> 6
<211> 1848
<212> DNA/RNA
<213> Homo sapiens

<400> 6
cgggcccggc cgggccttca gcggtttccc tgcagcctcc acgcgcccag gacccggctc 60
ccggcccccg cggtcccgct gagagccgag gcctcctttc ctcctcctgc ggctccggcg 120
acgccacctg atgcctccgc cgcccgcccg tccgccccgc gcggtcccct gggctcgcct 180
cggaggcggc gccggggacc ccgggctctg tctctgggcc ctccccggcg cgcccccgcc 240
ggctcccgag gccggagacc ttgaagagcg cggcgtcctc ctcgcggttg tagtcctgct 300
tgcccgcgtg cttccagggg atgcggaaga tgctcttctc ctcgttctcc cacaccagcc 360
cggggtactt gccgctgtcg atctggtcga tcagccactg gcggagcttc ccgttgccgc 420
agctcaccgc gctcatgccg aactctccgc ctcggccgcc gccctccagg ttcatgcccc 480
gcgtgccgtc cgcgcccgcg cccggggtcc tccgcccgct ctgctctgca ctgcggggag 540
aagagctgcc cttcagccac gaggcaccgc actccggcca ccccgccccg atcccctgcg 600
ccagtgacca cgaggccccg gagtctttga ggctgcgaag cgcgcgcgtg ccgtgtcagg 660
gtcgtccggg cccccggaag aaggcacctt cgcggccggc ccgggcgcat cccctgggcg 720
ggcacagccg tccgccttcc gagctcggcc cccgcgaagg ctcctacccg cctgctctcc 780
tgtaacgcac ccgcagccca aaggctcccg acacgcgccg ctcgtcttgc accctctctc 840
cccacccagc cgccacccct cctggccctc cttccgcact cactcgcagc tctcgcttgc 900
cttcgctcac tcccaacccg cttctcgccc cttccccgcg actctgtcag tttccactcc 960
cctggcggcc tccacgcgcg gaggacctcg cctgcggccg ggcgttccag gggcgcagcc 1020
ccgggttcct ccaccctccg ctttctctgc tcgcatccag acccttcgcc ccgaatagga 1080
cccctatccc tgcccagcta gcgcgcacta agtgggctct gcaaacgaca agtggcgcag 1140
acgcgggggc tgcagcccca gtacggggga ttccgcgcgc agagcgtccg ccggaccccg 1200
gagcaggccc gggagagcgg aggcggggag ggcgcgggag agggcgacga cagctgcgga 1260
gcctgggagg cagggaccgc gccaggccgg ctaggccacc gcacgcaagg ctcggagcga 1320
cccaaatgtg gagctcctcc tcctcctgcg ccagcgggtg agcaccgcgc cggaggccgc 1380

cgtgagcttg ggccccttag gaacgaagag caggggaagg agcctcggat tggggtccac 1440
agggccgggc ctgtttctcg caggcagttg tgccgtcttg tgtgggtgcc ttggacggcc 1500
ccgcctcagc cactcctggg gccccgacag tccggttagc tcatcccgtc cagcttgtgg 1560
cgaccccgtc gcaggagcgc ggagggcagg cggggaggct cgggcagagc caaggaggcg 1620
ggccttggag cccaaaccag cgcctccggc tcagcgcaga tggggtggga gtcggagctc 1680
caggacgggg cgcgctccct tcccgctccg aagccggggt accctgcgct gccctggagg 1740
tcgaacctct ggttcgcgct ccgggtcctc tctggtatca gcctcacacc cctcctcctg 1800
cccgactcca gcccttacct cgccctggac tcggagctga gggcagcg 1848

<210> 7
<211> 489
<212> DNA/RNA
<213> Artificial Sequence

<220>
<221> misc_feature
<222> (1)..(489)
<223> bisulfite treated sequence of SEQ ID NO:5


<220>
<221> misc_feature
<222> (1)..(489)
<223> y is c or u


<400> 7
yggggygggg yguygagguu tgtggggguy gggggttgga utggggagag ggautttgua 60
aguygagagu ygggguyggu ygggygggyg aguutuaggg uyggygtgaa ggutgggggu 120
tguygygguy ygggtggggt tuuygyguuu ttggygauuu tuygygyguy gyguuyggag 180
agyguauutu uaguygggyg ygyguygutt yggggautgt uautgggguy gtttuuygyg 240
aagggygagg tgygaaatgy gaatutyguu tttgyguuag gauutuagga gguuagtuaa 300
tuautaaaut guagygatgt gguuagggyg ggaaatgggg ggygtgtagt agygggaatu 360
tggtgygaag gggauttygu tttguagagy gtgtaaygga agayggagga atggtuuaaa 420
gagaautgga ggtggaggtt gygggaguau uutygguaag guuatuauyg ggaauuutgu 480
ututuygyg 489

<210> 8
<211> 1848
<212> DNA/RNA
<213> Artificial Sequence

<220>
<221> misc_feature
<222> (1)..(1848)
<223> bisulfite treated sequence of SEQ ID NO:6


<220>
<221> misc_feature
<222> (1)..(1848)
<223> y is c or u


<400> 8
yggguuyggu yggguuttua gyggtttuuu tguaguutuu aygyguuuag gauuyggutu 60
uygguuuuyg yggtuuygut gagaguygag guutuutttu utuutuutgy ggutuyggyg 120
ayguuauutg atguutuygu yguuyguuyg tuyguuuygy gyggtuuuut gggutyguut 180
yggaggyggy guyggggauu uygggututg tututggguu utuuuyggyg yguuuuyguy 240
ggutuuygag guyggagauu ttgaagagyg yggygtuutu utygyggttg tagtuutgut 300
tguuygygtg uttuuagggg atgyggaaga tgututtutu utygttutuu uauauuaguu 360
yggggtautt guygutgtyg atutggtyga tuaguuautg gyggaguttu uygttguygu 420
agutuauygy gutuatguyg aaututuygu utygguyguy guuutuuagg ttuatguuuy 480

gygtguygtu ygyguuygyg uuyggggtuu tuyguuygut utgututgua utgyggggag 540
aagagutguu uttuaguuay gagguauygu autuygggua uuuyguuuyg atuuuutgyg 600
uuagtgauua ygagguuuyg gagtutttga ggutgygaag ygygygygtg uygtgtuagg 660
gtygtuyggg uuuuyggaag aagguauutt ygygguyggu uygggyguat uuuutgggyg 720
gguauaguyg tuyguuttuy gagutygguu uuygygaagg utuutauuyg uutgututuu 780
tgtaayguau uyguaguuua aaggutuuyg auaygyguyg utygtuttgu auuutututu 840
uuuauuuagu yguuauuuut uutgguuutu uttuyguaut uautyguagu tutyguttgu 900
uttygutuau tuuuaauuyg uttutyguuu uttuuuygyg aututgtuag tttuuautuu 960
uutggygguu tuuaygygyg gaggauutyg uutgygguyg ggygtttuag gggyguaguu 1020
uygggttuut uuauuutuyg utttututgu tyguatuuag auuuttyguu uygaatagga 1080
uuuutatuuu tguuuaguta gygyguauta agtgggutut guaaaygaua agtggyguag 1140
aygygggggu tguaguuuua gtayggggga ttuygygygu agagygtuyg uyggauuuyg 1200
gaguagguuy gggagagygg aggyggggag ggygygggag agggygayga uagutgygga 1260
guutgggagg uagggauygy guuagguygg utagguuauy guayguaagg utyggagyga 1320
uuuaaatgtg gagutuutuu tuutuutgyg uuagygggtg aguauygygu yggagguygu 1380
ygtgaguttg gguuuuttag gaaygaagag uaggggaagg aguutyggat tggggtuuau 1440
aggguygggu utgtttutyg uagguagttg tguygtuttg tgtgggtguu ttggaygguu 1500
uyguutuagu uautuutggg guuuygauag tuyggttagu tuatuuygtu uaguttgtgg 1560
ygauuuygty guaggagygy ggaggguagg yggggaggut yggguagagu uaaggaggyg 1620
gguuttggag uuuaaauuag yguutuyggu tuagyguaga tggggtggga gtyggagutu 1680
uaggaygggg ygygutuuut tuuygutuyg aaguyggggt auuutgygut guuutggagg 1740
tygaauutut ggttygygut uygggtuutu tutggtatua guutuauauu uutuutuutg 1800
uuygautuua guuuttauut yguuutggau tyggagutga ggguagyg 1848

<210> 9
<211> 27
<212> DNA/RNA
<213> Primer

<400> 9
ttagtgagtg ygatgttttt taaatat 27

<210> 10
<211> 29
<212> DNA/RNA
<213> Primer

<400> 10
ataaaactct ctaaaacraa acctaaaac 29

<210> 11
<211> 32
<212> DNA/RNA
<213> Primer

<400> 11
gtatttttag ttttatygtt ygattttggg at 32

<210> 12
<211> 28
<212> DNA/RNA
<213> Primer

<400> 12
ccaaatataa aactcctcct cctcctac 28

<210> 13
<211> 29
<212> DNA/RNA
<213> Primer

<400> 13
taggaggagg aggagtttta tatttgggt 29

<210>  14
<211>  31
<212>  DNA/RNA
<213>  Primer

<400>  14
aaccacraaa ccccraaatc tttaaaacta c                                    31

<210>  15
<211>  34
<212>  DNA/RNA
<213>  Primer

<400>  15
   gtgttttttt tygggggtty ggaygatttt gata                                   34

<210>  16
<211>  30
<212>  DNA/RNA
<213>  Primer

<400>  16
aacccctcaa acctttaacc rcccttcccc                                      30

<210>  17
<211>  26
<212>  DNA/RNA
<213>  Primer

<400>  17
   gatttttagg gtagygtagg gtattt                                           26

<210>  18
<211>  30
<212>  DNA/RNA
<213>  Primer

<400>  18
caaataaaat aaaaatcraa actccaaaac                                      30

<210>  19
<211>  26
<212>  DNA/RNA
<213>  Primer

<400>  19
   gatttttagg gtagygtagg gtattt                                           26

<210>  20
<211>  45
<212>  DNA/RNA
<213>  Primer

<400>  20
yggtttygga gygggaaggg agygygttty gttttggagt ttyga                     45

## Claims

1. An isolated polynucleotide, wherein the isolated polynucleotide comprises:
(a) a polynucleotide with a nucleotide sequence as shown in SEQ ID NO: 1;
(b) a polynucleotide with a nucleotide sequence as shown in SEQ ID NO: 2;
(c) a fragment of the polynucleotide of (a)-(b), having at least one CpG site with modification;
(d) a nucleic acid complementary to the polynucleotide or fragment of (a)-(c).

2. The isolated polynucleotide according to claim 1, wherein the modification comprises 5-methylation, 5-hydroxymethylation, 5-formylcytosine or 5-carboxylcytosine.

3. An isolated polynucleotide, wherein, the polynucleotide is converted from the polynucleotide according to claim 1 or 2, and as compared with the sequence in claim 1, the cytosine C of the CpG site(s) with modification is unchanged, and the unmodified cytosine is converted into T or U.

4. The polynucleotide according to claim 3, wherein the polynucleotide comprises:
(e) the polynucleotide with a nucleotide sequence as shown in SEQ ID NO: 3 or 7;
(f) the polynucleotide with a nucleotide sequence as shown in SEQ ID NO: 4 or 8;
(g) a fragment of the polynucleotide of (e)-(f), having at least one CpG site with modification.

5. Use of the polynucleotide according to any of claims 1-4 in manufacture of a tumor detection agent or kit.

6. The use according to claim 5, wherein, the tumors comprise: digestive system tumors such as esophageal cancer, gastric cancer, colorectal cancer, liver cancer, pancreatic cancer, bile duct and gallbladder cancer; gynecological and reproductive system tumors such as breast cancer, ovarian cancer, cervical cancer, vulvar cancer, testicular cancer, prostate cancer, penile cancer; hematologic cancers such as leukemia, lymphoma, multiple myeloma; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

7. The use according to claim 5 or 6, wherein samples of the tumor comprise: tissue samples, paraffin embedded samples, blood samples, pleural effusion samples, alveolar lavage fluid samples, ascites and lavage fluid samples, bile samples, stool samples, urine samples, saliva samples, sputum samples, cerebrospinal fluid samples, cell smear samples, cervical scraping or brushing samples, tissue and cell biopsy samples.

8. A method of preparing a tumor detection agent, comprising: providing the polynucleotide according to any of claims 1-4, designing a detection agent for specifically detecting the modification on CpG(s) of the target sequence which is the full length or fragment of the polynucleotide; wherein, the target sequence has at least one modified CpG site.

9. An agent or a combination of agents, wherein, the agent or the combination of agents specifically detect the modification on CPG site(s) of a target sequence, which is the full length or fragment of the polynucleotides according to any of claims 1-4 and has at least one modified CpG site.

10. The agent or the combination of agents according to claim 9, wherein the agent or combination of agents is for a gene sequence containing the target sequence, and the gene sequence comprises gene Panels or gene groups.

11. The agent or the combination of agents according to claim 9, wherein the agent or combination of agents comprise: primers or probes; preferably, the primers are selected from the group consisting of: the primers shown in SEQ ID NO: 9 and 10; the primers shown in SEQ ID NO: 11 and 12; the primers shown in SEQ ID NO: 13 and 14; the primers shown in SEQ ID NO: 15 and 16; the primers shown in SEQ ID NO: 17 and 18.

12. Use of the agent or combination of agents according to any of claims 9-11 in the manufacture of a kit for detecting tumors; preferably, the tumors comprise: digestive system tumors such as esophageal cancer, gastric cancer, colorectal cancer, liver cancer, pancreatic cancer, bile duct and gallbladder cancer; gynecological and reproductive system tumors such as breast cancer, ovarian cancer, cervical cancer, vulvar cancer, testicular cancer, prostate cancer, penile cancer; hematologic cancers such as leukemia, lymphoma, multiple myeloma; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

13. A detection kit, comprising:
container(s) and the agent or combination of agents according to any of claims 9-11 in the container(s).

14. A method of detecting the methylation profile of a sample *in vitro,* comprising:
(i) providing the sample and extracting nucleic acid;
(ii) detecting modification on CPG site(s) of a target sequence in the nucleic acid of (i), wherein the target sequence is the polynucleotide according to claim 1 or 2 or the polynucleotide according to claim 3 or 4 that converted from the polynucleotide according to claim 1 or 2.

15. The method according to claim 14, wherein, in step (3), a analysis method comprise pyrosequencing, bisulfite conversion sequencing, a method using methylation chip, qPCR, digital PCR, second generation sequencing, third generation sequencing, whole genome methylation sequencing, DNA enrichment detection, simplified bisulfite sequencing technology, HPLC, MassArray, methylation specific PCR, or their combination, as well as *in vitro* detection and *in vivo* tracer detection for the combined gene group of partial or all of the methylation sites in the sequence shown in SEQ ID NO: 1 or 2.

16. The method according to claim 14, wherein, step (ii) comprises:
(1) treating the product of (i) to convert unmodified cytosine into uracil; preferably, the modification includes 5-methylation, 5-hydroxymethylation, 5-formylcytosine or 5-carboxylcytosine; preferably, treating the nucleic acid of step (i) with bisulfite;
(2) analyzing the modification of the target sequence in the nucleic acid treated by (1).
